# EUROPÄISCHE PATENTANMELDUNG

(11) **EP 3 524 236 A1**
(43) Veröffentlichungstag der Anmeldung: **14.08.2019**
(21) Anmeldenummer: 19160989.0
(22) Anmeldetag: 16.08.2004
(51) Int. Cl.: A61K 31/12, A61P 17/14, A61P 17/04, A61P 17/08, A61K 8/97, A61Q 19/00, A61Q 17/04, A61Q 7/00, A61Q 19/08, A61K 8/35

(54) **VERWENDUNG VON LICOCHALCHON A ODER EINES LICOCHALCHON A ENTHALTENDEN EXTRAKTES AUS RADIX GLYCYRRHIZAE INFLATAE GEGEN HAUTALTERUNG**

(30) Priorität: 12.09.2003 DE 10342212
(62) Teilanmeldung aus: 04764150.1
(71) Anmelder: Beiersdorf AG, 20253 Hamburg (DE)
(72) Erfinder: Breitenbach, Ute, 22299 Hamburg (DE); Gallinat, Stefan, 22880 Hamburg (DE); Kolbe, Ludger, 21255 Dohren (DE); Mummert, Christian, 29553 Bienenbüttel (DE); Blatt, Thomas, 22880 Wedel (DE); Wolber, Rainer, 22397 Hamburg (DE); Stäb, Franz, 21379 Echem (DE)

(57) **Zusammenfassung**

Verwendung von Licochalcon A oder eines Licocalchon A enthaltenden Extraktes aus Radix Glycyrrhizae inflatae in kosmetischen oder dermatologischen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut.

## Beschreibung

Die vorliegende Erfindung betrifft kosmetische bzw. dermatologische dermatologische Zubereitungen, enthaltend Wirkstoffe zur Pflege und zum Schutze der Haut, insbesondere der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Diesen Übelständen abzuhelfen, war Aufgabe der vorliegenden Erfindung.

Es hat sich überraschenderweise herausgestellt, dass die Verwendung von Licochalcon A oder eines Licocalchon A enthaltenden Extraktes aus Radix Glycyrrhizae inflatae in kosmetischen oder dermatologischen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut den Nachteilen das Standes der Technik abhilft.

Bei Anwendung des erfindungsgemäß verwendeten Wirkstoffes bzw. kosmetischer oder topischer dermatologischer Zubereitungen mit einem wirksamen Gehalt an erfindungsgemäß verwendetem Wirkstoff ist in überraschender Weise eine wirksame Behandlung, aber auch eine Prophylaxe
- von defizitären, oder hypoaktiven Hautzuständen oder defizitären, oder hypoaktiven Zustände von Hautanhangsgebilden
- von Erscheinungen vorzeitiger Alterung der Haut (z.B. Falten, Altersflecken, Teleangiektasien) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde.
- von lichtbedingten Hautschäden,
- von Juckreiz,
- von trockenen Hautzuständen und Hornschichtbarrierestörungen,
- von Haarausfall und für verbessertes Haarwachstum
- von entzündlichen Hautzuständen wie dem seborrhoischem Ekzem, polymorpher Lichtdermatose und Vitiligo
   möglich. Der erfindungsgemäße Wirkstoffes bzw. kosmetischer oder topischer dermatologischer Zubereitungen mit einem wirksamen Gehalt an erfindungsgemäßem Wirkstoff dient aber auch in überraschender Weise
- zur Stimulation der Kollagen-, Hyaluronsäure-, Elastinsynthese
- zur Stimulation der intrazellulären DNA-Synthese, insbesondere bei defizitären oder hypoaktiven Hautzuständen.
- zur Steigerung der Zellerneuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Es ist erfindungsgemäß insbesondere äußerst vorteilhaft, den erfindungsgemäß verwendeten Wirkstoff bzw. kosmetische oder topische dermatologische Zubereitungen mit einem wirksamen Gehalt an erfindungsgemäß verwendetem Wirkstoff zur kosmetischen oder dermatologischen Behandlung oder Prophylaxe unerwünschter Hautzustände zu verwenden.

Vorteilhaft ist insbesondere eine erfindungsgemäße Verwendung, dadurch gekennzeichnet, dass die Zubereitungen 0,0001 bis 5 Gew.-%, insbesondere 0,001 bis 1 Gew.-%, ganz besonders 0,005 bis 0,15 Gew.-% Licochalcon A enthalten, bezogen auf das Gesamtgewicht der Zubereitung.

Vorteilhaft ist ferner insbesondere eine erfindungsgemäße Verwendung, dadurch gekennzeichnet, dass die Zubereitungen 0,001 bis 10 Gew.-%, insbesondere 0,05 bis 5 Gew.-%, ganz besonders 0,01 bis 2 Gew.-% an einem oder mehreren Polyolen enthalten, bezogen auf das Gesamtgewicht der Zubereitung.

Vorteilhaft ist ferner insbesondere eine erfindungsgemäße Verwendung, dadurch gekennzeichnet, dass die Zubereitungen Licocalchon als Bestandteil von pflanzlichen Extrakten, insbesondere von *Radix Glycyrrhizae inflatae,* enthalten.

Die Pflanzenart *Glycyrrhiza inflata* gehört wie das in Europa offizielle Süßholz *Glycyrrhiza glabra* der Gattung *Glycyrrhiza* an, die zur Pflanzenfamilie der *Fabaceae* (Erbsengewächse) gehört. Die Droge *Radix Glycyrrhizae inflatae,* d.h., die Wurzel der Pflanze, ist, beispielsweise in der fernöstlichen Medizin, gebräuchlich. Die Verwendung der Droge als Entzündungshemmer ist ebenfalls bekannt.

Ein Bestandteil des wässrigen Auszugs aus Radix Glycyrrhizae inflatae ist das Licochalcon A, welches sich durch die folgende Strukturformel auszeichnet:

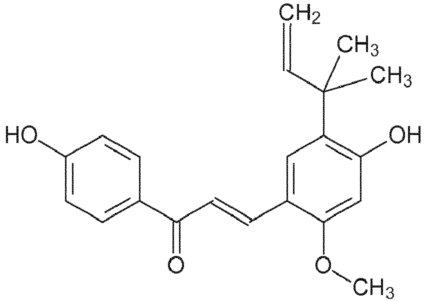

Es wird angenommen, dass diese Substanz, möglicherweise in Synergie mit den übrigen Bestandteilen des Extraktes, Anteil an der erfindungsgemäßen Wirkung besitzt.

Erfindungsgemäß ist demnach auch die Verwendung einer Kombination aus
- Licochalcon A
- Wasser
- gegebenenfalls einem oder mehreren Polyolen.
in kosmetischen oder dermatologischen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.-% an einem wässrigen Extrakt aus *Radix Glycyrrhizae inflatae* enthalten, bezogen auf das Gesamtgewicht der Zubereitung.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.-% an einem oder mehreren ethoxylierten oder propoxylierten Rohstoffen enthalten, bezogen auf das Gesamtgewicht der Zubereitung.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.-% an einem oder mehreren Polyolen enthalten, bezogen auf das Gesamtgewicht der Zubereitung.

Insbesondere ist vorteilhaft, als Polyol das Butylenglycol zu wählen.

Ganz besonders vorteilhaft ist es, von einem Extrakt auszugehen, der unter der Bezeichnung Polyol Soluble Licorice Extract P-U von der Firma Maruzen vertrieben wird.

Ferner ist es vorteilhaft, Licochalcone A in anderen Vehikelsystemen in einer Konzentration von 0,0001 bis 5 Gew.-%, insbesondere 0,001 bis 1 Gew.-%, ganz besonders 0,005 - 0,05 Gew.-% zu verwenden.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, dass der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-ÖI (W/O) oder vom Typ Öl-in-Wasser (O/W), oder eine multiple Emulsionen, beispielsweise vom Typ Wasser-in-ÖI-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wässrige Systeme bzw. Tensidzubereitungen zur Reinigung der Haut und der Haare einzufügen.

Es ist dem Fachmanne natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Günstig sind gegebenenfalls auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoesäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wässrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, CelluloseDerivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele O/W-Crèmes

**Beispiel Nr. 1**

| | |
|---|---|
| Glycerylsterat selbstemulgierend | 4,00 |
| PEG-40-Stearat | 1,00 |
| Cetylalkohol | 3,00 |
| Caprylic-/Capric-Triglycerid | 5,00 |
| Paraffinum liquidum | 5,00 |
| Polyol Soluble Licorice Extract P-U | 0,025 |
| Tocopherol | 0,1 |
| Na₃HEDTA | 0,1 |
| Konservierungsmittel, Parfum | q.s. |
| Polyacrylsäure | 3,00 |
| Natronlauge 45% | q.s |
| Glycerin | 5,00 |
| Wasser | ad100 |

**Beispiel Nr. 2**

| | |
|---|---|
| Glycerylsterat selbstemulgierend | 3,00 |
| Stearinsäure | 1,00 |
| Cetylalkohol | 2,00 |
| Caprylic-/Capric-Triglycerid | 3,00 |
| Dicaprylylether | 4,00 |
| Paraffinum liquidum | 2,00 |
| Polyol Soluble Licorice Extract P-U | 0,05 |
| Konservierungsmittel, Parfum | q.s. |
| Polyacrylsäure | 0,1 |
| Natronlauge 45% | q.s. |
| Glycerin | 3,00 |
| Butylenglycol | 3,00 |
| Wasser | ad100 |

**Beispiel Nr. 3**

| | |
|---|---|
| Glycerylstearatcitrat | 2,00 |
| Stearylalkohol | 2,00 |
| Lanolinalkohol | 1,00 |
| Caprylic-/Capric-Triglycerid | 4,00 |
| Paraffinum liquidum | 8,00 |
| Dimethicon | 1,00 |
| Licochalcon A | 0,025 |
| Konservierungsmittel, Parfum | q.s. |
| Natronlauge 45% | q.s. |
| Glycerin | 7,50 |
| Wasser | ad100 |

**Beispiel Nr. 4**

| | |
|---|---|
| Glycerylstearatcitrat | 2,00 |
| Stearylalkohol | 2,00 |
| Lanolinalkohol | 1,00 |
| Caprylic-/Capric-Triglycerid | 4,00 |
| Paraffinum liquidum | 8,00 |
| Dimethicon | 1,00 |
| Polyol Soluble Licorice Extract P-U | 0,15 |
| Konservierungsmittel, Parfum | q.s. |
| Natronlauge 45% | q.s. |
| Glycerin | 7,50 |
| Dihydroxyaceton | 1,00 |
| Wasser | ad100 |

**Beispiel Nr. 5**

| | |
|---|---|
| Polyglyceryl-3-Methylglucose-Distearat | 3,00 |
| Cetylalkohol | 3,00 |
| Caprylic-/Capric-Triglycerid | 3,00 |
| Dicaprylylether | 2,00 |
| Paraffinum liquidum | 3,00 |
| Polyol Soluble Licorice Extract P-U | 1,00 |
| Na3HEDTA | 0,1 |
| Konservierungsmittel, Parfum | q.s. |
| Polyacrylsäure | 0,1 |
| Natronlauge 45% | q.s. |
| Glycerin | 3,00 |
| Wasser | ad100 |

**Beispiel Nr. 6**

| | |
|---|---|
| Glycerylstearatcitrat | 2,00 |
| Sorbitanstearat | 2,00 |
| Cetylstearylalkohol | 2,00 |
| Caprylic-/Capric-Triglycerid | 3,00 |
| Octyldodecanol | 2,00 |
| Dicaprylylether | 1,00 |
| Polyol Soluble Licorice Extract P-U | 0,05 |
| Tocopherol | 0,20 |
| Konservierungsmittel, Parfum | q.s. |
| Polyacrylsäure | 0,1 |
| Natronlauge 45% | q.s. |
| Glycerin | 3,00 |
| Wasser | ad100 |

### Beispiele O/W-Cremes

**Beispiel Nr. 7**

| | |
|---|---|
| Glycerylsterat selbstemulgierend | 5,00 |
| Stearylalkohol | 2,00 |
| Caprylic-/Capric-Triglycerid | 2,00 |
| Octyldodecanol | 2,00 |
| Dimethicon Polydimethylsiloxan | 2,00 |
| Titandioxid | 2,00 |
| 4-Methylbenzylidencampher | 1,00 |
| Butylmethoxy-dibenzoylmethan | 0,50 |
| Licochalcone A | 0,08 |
| Konservierungsmittel, Parfum | q.s. |
| Polyacrylsäure | 0,15 |
| Natronlauge 45% | q.s. |
| Glycerin | 3,00 |
| Wasser | ad 100 |

**Beispiel Nr. 8**

| | |
|---|---|
| Glycerylstearatcitrat | 2,00 |
| Cetylstearylalkohol | 3,00 |
| C12-15 Alkylbenzoat | 2,00 |
| Octyldodecanol | 2,00 |
| Paraffinum liquidum | 4,00 |
| Polyol Soluble Licorice Extract P-U | 0,50 |
| 2,4-Bis-(4-(2-ethyl-hexyloxy-)2-hydroxyl)-phenyl)-6-(4-methoxyphenyl)-(1,3,5)-triazin | 1,0 |
| Dihydroxyaceton | 0,5 |
| Konservierungsmittel, Parfum | q.s. |
| Polyacrylsäure | 0,1 |
| Natronlauge 45% | q.s. |
| Butylenglycol | 3,00 |
| Ethanol | 3,00 |
| Wasser | ad 100 |

**Beispiel Nr. 9**

| | |
|---|---|
| Glycerylstearatcitrat | 2,00 |
| Cetylstearylalkohol | 1,00 |
| C12-15 Alkylbenzoat | 3,00 |
| Paraffinum liquidum | 2,00 |
| Polyol Soluble Licorice Extract P-U | 0,1 |
| 2,4-Bis-(4-(2-ethyl-hexyloxy-)2-hydroxyl)-phenyl)-6-(4-methoxyphenyl)-(1,3,5)-triazin | 3,0 |
| Ethylendiamintetraessigsäure Trinatrium | 0,20 |
| Konservierungsmittel, Parfum | q.s. |
| Xanthan Gummi | 0,20 |
| Natronlauge 45% | q.s. |
| Glycerin | 3,00 |
| Wasser | ad 100 |

**Beispiel Nr. 10**

| | |
|---|---|
| Stearinsäure | 2,50 |
| Cetylalkohol | 3,00 |
| Octyldodecanol | 4,00 |
| Cyclisches Dimethylpolysiloxan | 0,50 |
| Polyol Soluble Licorice Extract P-U | 1,00 |
| Konservierungsmittel, Parfum | q.s. |
| Polyacrylsäure | 0,05 |
| Natronlauge 45% | q.s. |
| Glycerin | 5,00 |
| Ethanol | 3,00 |
| Wasser | ad 100 |

**Beispiel Nr. 11**

| | |
|---|---|
| Stearinsäure | 3,50 |
| Cetylalkohol | 4,50 |
| Cetylstearylalkohol | 0,50 |
| Octyldodecanol | 6,00 |
| Cyclisches Dimethylpolysiloxan | 2,00 |
| 4-Methylbenzylidencampher | 1,00 |
| Butylmethoxy-dibenzoylmethan | 0,50 |
| Polyol Soluble Licorice Extract P-U | 0,40 |
| 2,4-Bis-(4-(2-ethyl-hexyloxy-)2-hydroxyl)-phenyl)-6-(4-methoxyphenyl)-(1,3,5)-triazin | 0,5 |
| Dihydroxyaceton | 0,5 |
| Tocopherol | 0,05 |
| Ethylendiamintetraessigsäure Trinatrium | 0,20 |
| Konservierungsmittel, Parfum | q.s. |
| Polyacrylsäure | 0,05 |
| Natronlauge 45% | q.s. |
| Glycerin | 3,00 |

### Beispiele W/O-Emulsionen

**Beispiel Nr. 12**

| | |
|---|---|
| Polyglyceryl-2-dipolyhydroxystearat | 5,00 |
| 2,4-Bis-(4-(2-ethyl-hexyloxy-)2-hydroxyl)-phenyl)-6-(4-methoxyphenyl)-(1,3,5)-triazin | 2,00 |
| Diethylhexyl Butamidotriazon | 3,00 |
| Octocrylen | 7,00 |
| Diethylhexyl Butamidotriazon | 1,00 |
| Phenylen-1,4-bis-(mononatrium,-2-benzimidazyl-5,7-disulfonsäure) | 1,00 |
| Phenylbenzimidazol Sulfonsäure | 0,50 |
| Zinkoxid | 3,00 |
| Dicaprylylether | 10,00 |
| Dicaprylylcarbonat | 5,00 |
| Phenylmethylpolysiloxan | 2,00 |
| PVP Hexadecencopolymer | 0,50 |
| Glycerin | 3,00 |
| Magnesiumsulfat | 1,00 |
| Tocopherolacetat | 0,50 |
| Polyol Soluble Licorice Extract P-U | 0,15 |
| Konservierungsmittel, Parfum | q.s. |
| Ethanol | 3,00 |
| Wasser | ad 100 |

**Beispiel Nr. 13**

| | |
|---|---|
| Cetyldimethiconcopolyol | 2,50 |
| 2-Ethylhexyl Methoxyzinnamat | 8,00 |
| 2,4-Bis-(4-(2-ethyl-hexyloxy-)2-hydroxyl)-phenyl)-6-(4-methoxyphenyl)-(1,3,5)-triazin | 2,50 |
| Diethylhexyl Butamidotriazon | 1,00 |
| 4-Methylbenzylidencampher | 2,00 |
| Octocrylen | 2,50 |
| Phenylen-1,4-bis-(mononatrium,-2-benzimidazyl-5,7-disulfonsäure) | 2,00 |
| Titandioxid | 2,00 |
| Zinkoxid | 1,00 |
| Dimethicon Polydimethylsiloxan | 4,00 |
| Phenylmethylpolysiloxan | 25,00 |
| Octoxyglycerin | 0,30 |
| Glycerin | 7,50 |
| Glycinsoja | 1,00 |
| Magnesiumsulfat | 0,50 |
| Polyol Soluble Licorice Extract P-U | 0,08 |
| Konservierungsmittel, Parfum | q.s. |
| Wasser | ad 100 |

**Beispiel Nr. 14**

| | |
|---|---|
| PEG-30-dipolyhydroxystearat | 5,00 |
| Butylmethoxy-dibenzoylmethan | 2,00 |
| Ethylhexyl Triazon | 3,00 |
| Octocrylen | 4,00 |
| Phenylen-1,4-bis-(mononatrium,-2-benzimidazyl-5,7-disulfonsäure) | 0,50 |
| Titandioxid | 1,50 |
| Zinkoxid | 2,00 |
| Paraffinum liquidum | 10,0 |
| Butylen-Glycol-Dicaprylat/-Dicaprat | 2,00 |
| Dicaprylylcarbonat | 6,00 |
| Dimethicon Polydimethylsiloxan | 1,00 |
| Shea Butter | 3,00 |
| Octoxyglycerin | 1,00 |
| Glycinsoja | 1,50 |
| Magnesiumchlorid | 1,00 |
| Tocopherolacetat | 0,25 |
| Polyol Soluble Licorice Extract P-U | 0,5 |
| Konservierungsmittel, Parfum | q.s. |
| Ethanol | 1,50 |
| Wasser | ad 100 |

**Beispiel Nr. 15**

| | |
|---|---|
| Cetyldimethiconcopolyol | 4,00 |
| 2-Ethylhexyl Methoxyzinnamat | 5,00 |
| 2,4-Bis-(4-(2-ethyl-hexyloxy-)2-hydroxyl)-phenyl)-6-(4-methoxyphenyl)-(1,3,5)-triazin | 2,00 |
| Butylmethoxy-dibenzoylmethan | 1,00 |
| Ethylhexyl Triazon | 4,00 |
| 4-Methylbenzylidencampher | 4,00 |
| Diethylhexyl Butamidotriazon | 2,00 |
| Phenylbenzimidazol Sulfonsäure | 3,00 |
| Zinkoxid | 0,50 |
| C₁₂₋₁₅ Alkyl-Benzoat | 9,00 |
| Butylen-Glycol-Dicaprylat/-Dicaprat | 8,00 |
| Dimethicon Polydimethylsiloxan | 5,00 |
| PVP Hexadecencopolymer | 0,50 |
| Glycerin | 7,50 |
| Magnesiumsulfat | 0,50 |
| Polyol Soluble Licorice Extract P-U | 1,00 |
| Konservierungsmittel, Parfum | q.s. |
| Wasser | ad 100 |

**Beispiel Nr. 16**

| | |
|---|---|
| Polyglyceryl-2-dipolyhydroxystearat | 4,50 |
| 2-Ethylhexyl Methoxyzinnamat | 4,00 |
| 2,4-Bis-(4-(2-ethyl-hexyloxy-)2-hydroxyl)-phenyl)-6-(4-methoxyphenyl)-(1,3,5)-triazin | 2,50 |
| Diethylhexyl Butamidotriazon Ethylhexyl Triazon | 3,00 |
| 4-Methylbenzylidencampher | 2,00 |
| Octocrylen | 2,50 |
| Phenylbenzimidazol Sulfonsäure | 2,00 |
| Titandioxid | 3,00 |
| Paraffinum liquidum | 8,00 |
| Dicaprylylether | 7,00 |
| Butylen-Glycol-Dicaprylat/-Dicaprat | 4,00 |
| Phenylmethylpolysiloxan | 2,00 |
| PVP Hexadecencopolymer | 1,00 |
| Octoxyglycerin | 0,50 |
| Glycerin | 2,50 |
| Magnesiumchlorid | 0,70 |
| Tocopherolacetat | 1,00 |
| Polyol Soluble Licorice Extract P-U | 0,80 |
| Konservierungsmittel, Parfum | q.s. |
| Ethanol | 1,00 |
| Wasser | ad 100 |

### Beispiele W/O Emulsionen

| **Beispiel Nr.** | **17** | **18** |
|---|---|---|
| Polyglyceryl-2-dipolyhydroxystearat | 4,00 | 5,00 |
| Lanolinalkohol | 0,50 | 1,50 |
| Isohexadecan | 1,00 | 2,00 |
| Myristyl-Myristat | 0,50 | 1,50 |
| Vaseline | 1,00 | 2,00 |
| Butylmethoxy-dibenzoylmethan | 0,50 | 1,50 |
| 4-Methylbenzylidencampher | 1,00 | 3,00 |
| Butylen-Glycol-Dicaprylat/-Dicaprat | 4,00 | 5,00 |
| Shea Butter | - | 0,50 |
| Butylenglycol | - | 6,00 |
| Octoxyglycerin | - | 3,00 |
| Glycerin | 5,00 | - |
| Tocopherolacetat | 0,50 | 1,00 |
| Polyol Soluble Licorice Extract P-U | 0,2 | 0,1 |
| EDTA | 0,20 | 0,20 |
| Konservierungsmittel | q.s. | q.s. |
| Ethanol | - | 3,00 |
| Parfum | q.s. | q.s. |
| Wasser | ad 100 | ad 100 |

### Beispiel (W/O-Creme)

**Beispiel Nr. 19**

| | |
|---|---|
| Polyglyceryl-3-Diisostearat | 3,50 |
| Glycerin | 3,00 |
| Polyglyceryl-2-Dipolyhydroxystearat | 3,50 |
| Polyol Soluble Licorice Extract P-U | 0,25 |
| Konservierungsmittel | q.s. |
| Parfum | q.s. |
| Magnesiumsulfat | 0,6 |
| Isopropylstearat | 2,0 |
| Caprylylether | 8,0 |
| Cetearylisononanoat | 6,0 |
| Wasser | ad 100 |

### Beispiel (W/O-Emulsion):

**Beispiel Nr. 20**

| | |
|---|---|
| Triceteareth-4-Phosphat | 0,80 |
| Butylhydroxytoluol | 0,05 |
| Glyceryllanolat | 1,70 |
| Cyclomethicon | 2,20 |
| Isopropylpalmitat | 1,00 |
| Polyol Soluble Licorice Extract P-U | 0,050 |
| Polyacrylsäure | 0,50 |
| Ethylendiamintetraessigsäure | 1,00 |
| Natriumhydroxid | q.s. |
| Zitronensäure | 0,01 |
| Konservierungsmittel | q.s. |
| Parfum | q.s. |
| Wasser | ad 100 |

## Patentansprüche

1. Verwendung von Licochalcon A oder eines Licocalchon A enthaltenden Extraktes aus Radix Glycyrrhizae inflatae in kosmetischen oder dermatologischen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut.

2. Verwendung nach Anspruch 1, **dadurch gekennzeichnet, dass** die Zubereitungen 0,0001 bis 5 Gew.-%, insbesondere 0,001 bis 1 Gew.-%, ganz besonders 0,005 bis 0,15 Gew.-% Licochalcon A enthalten, bezogen auf das Gesamtgewicht der Zubereitung.

3. Verwendung nach Anspruch 1, **dadurch gekennzeichnet, dass** die Zubereitungen 0,001 bis 10 Gew.-%, insbesondere 0,05 bis 5 Gew.-%, ganz besonders 0,01 bis 2 Gew.-% an einem oder mehreren Polyolen enthalten, bezogen auf das Gesamtgewicht der Zubereitung.

4. Verwendung nach Anspruch 1, **dadurch gekennzeichnet, dass** folgende Alterungsvorgänge oder -zustände der Haut behandelt oder ihnen vorgebeugt wird:
- defizitäre oder hypoaktive Hautzustände oder defizitäre oder hypoaktive Zustände von Hautanhangsgebilden
- Erscheinungen vorzeitiger Alterung der Haut (z.B. Falten, Altersflecken, Teleangiektasien) und/oder der Hautanhangsgebilde,
- umweltbedingte (Rauchen, Smog, reaktive Sauerstoffspecies, freie Radikale) und insbesondere lichtbedingte negative Veränderungen der Haut und der Hautanhangsgebilde.
- lichtbedingte Hautschäden
- Pigmentierungsstörungen,
- Juckreiz,
- trockenen Hautzuständen und Hornschichtbarrierestörungen,
- Haarausfall.
